# EUROPEAN PATENT APPLICATION

(11) **EP 4 241 749 A1**
(43) Date of publication of application: **13.09.2023**
(21) Application number: 22161478.7
(22) Date of filing: 10.03.2022
(51) Int. Cl.: A61F 13/26

(54) **TAMPON APPLICATOR**

(71) Applicant: Ontex BV, 9255 Buggenhout (BE); Ontex Group NV, 9320 Erembodegem (BE)
(72) Inventor: Matthes, Andreas, 02692 Grosspostwitz (DE); Lesage, Henri, 9900 Eeklo (BE); Sequeira Nogueira, Frederico Álvaro, 02625 Bautzen (DE)
(74) Representative: Saurat, Thibault

(57) **Abstract**

The invention concerns a tampon applicator (2) comprising at least three separate components selected from a barrel (4), a plunger (6) and a fingergrip (8), wherein :
- the barrel (4) is formed from a material comprising cellulosic fibers,
- the plunger (6) is formed from a material comprising cellulosic fibers, and
- the fingergrip (8) is formed from a material comprising a mixture of cellulosic fibers and starch.

The invention also pertains to an assembly comprising such a tampon applicator (2) and the use of a material comprising a mixture of cellulosic fibers and starch to form such a fingergrip.

## Description

### TECHNICAL FIELD

The invention pertains to the technical field of cardboard or other paper-based tampon applicators as well as a method to manufacture such tampon applicator as well as the use of a material comprising a mixture of cellulosic fibers and starch to form a fingergrip component of a tampon applicator.

### BACKGROUND

Tampon applicators are generally either a moulded thermoplastic material, such as plastic, or a cardboard or other paper-based materials, such as paperboard or paper laminate, collectively referred to herein as "cardboard".

Moulded plastic is often used to form a tampon applicator. Such applicators have a generally cylindrical shape with a lower expulsion force needed to eject the tampon pledget from the applicator. Furthermore, these moulded plastic applicators can be formed with a high degree of surface smoothness, which results in increased comfort during insertion of the tampon. However, these plastic tampon applicators are usually made from non-biodegradable and non-water dispersible material and thus are not environmentally friendly unless certain expensive plastics are used.

To that end, a variety of commercial tampon applicators appeared on the market which are formed from cardboard. However, these cardboard applicators are formed with two telescoping tubes and can be perceived by consumers as difficult to insert. Namely, these cardboard tampon applicators lack good griping features and are usually slippery.

Solutions have been suggested in the past where the cardboard applicator is coated with a plastic layer, for example publications such as US 5,931,803 or US 6,095,999 describe such solution. Although these disclosures describe a comfort of use, there is still a need to improve the environmental impact of these applicators and offer a solution that involves less plastic while maintaining good gripping features.

To that end, the invention aims to provide a tampon applicator that meets the standard performances of a plastic tampon applicator while maintaining a low environmental impact.

### SUMMARY OF THE INVENTION

The present invention concerns a tampon applicator comprising at least three separate components selected from a barrel, a plunger and a fingergrip, wherein the barrel is formed from a material comprising cellulosic fibers, the plunger is formed from a material comprising cellulosic fibers, and the fingergrip is formed from a material comprising a mixture of cellulosic fibers and starch.

The discrete barrel, fingergrip and plunger are all made from, or made out of, sustainable materials and the fingergrip has the benefit of being formed from a material that is more malleable, or flexible, than cardboard and thus can be processed, or molded, more easily to form fingergrips with specific shapes and/or specific features. The barrel, fingergrip and plunger each comprising a sustainable material make the tampon applicator more environmentally friendly.

According to an embodiment, the material comprising cellulosic fibers is cardboard or paper and/or the material comprising a mixture of cellulosic fibers and starch is a mixture of cardboard or paper and starch derived from natural sources such as corn, wheat, rice, tapioca, potato, and/or maize.

The starch is preferably industrial starch where the starch derived from natural sources is extracted and refined from seeds, roots and tubers, by processes such as wet grinding, washing, sieving and drying.

According to an embodiment, wherein the material forming the fingergrip comprises between 60 to 80 wt. % of starch, preferably between 65 to 75 wt.% of starch, more preferably about 70% of starch, and between 8 to 25 wt.% of cellulosic fibers, preferably between 10 to 20 wt.% of cellulosic fibers.

Such composition, and amounts in said composition, enables an easier processing, or molding, of the material to form fingergrips with specific shapes and/or specific features.

According to an embodiment, the fingergrip comprises a flange projecting from an outer surface of the fingergrip in a radial direction preferably beyond an outer surface of the barrel.

A flange, or ring or disc, forms a good gripping feature that can be easily formed during molding of the fingergrip.

According to an embodiment, the flange is arranged in a central portion of the fingergrip.

According to an embodiment, the fingergrip and the barrel comprise interlocking means connecting the fingergrip to the barrel.

According to an embodiment, the fringergrip comprises a raised portion comprising a plurality of raised elements such as ribs, embossments or ledges arranged on the exterior surface of the fingergrip.

According to an embodiment, the fingergrip comprises a thread arranged on the exterior surface of the fingergrip.

According to an embodiment, the barrel comprises a thread arranged on the interior surface of the barrel of complementary shape, and/or pitch, as the thread on the fingergrip.

According to an embodiment, the fingergrip comprises a tapered or curved portion arranged between the flange and one end of the fingergrip, wherein the outer diameter of the fingergrip decreases from the flange to said one end.

According to an embodiment, the fingergrip comprises the tapered or curved portion arranged on one side of the flange and a raised portion arranged on the opposite side of the flange.

According to an embodiment, the diameter of the flange 20 is at least 2 mm greater than the outer diameter of the barrel 4.

According to an embodiment, the fingergrip comprises a shouldered portion on the interior surface of the fingergrip.

According to an embodiment, the fingergrip has a different texture in comparison to the barrel or plunger.

The invention also pertains to an assembly comprising a tampon applicator according to any of the preceding claims and a tampon pledget comprising absorbent material arranged within the barrel of the tampon applicator.

The invention also pertains to the use of a material comprising a mixture of cellulosic fibers and starch to form a fingergrip component of a tampon applicator, said applicator comprising a circumferential flange for providing an environmentally friendly applicator with improved gripping features.

Further embodiments are described below and in the claims.

### DESCRIPTION OF FIGURES

The drawings and figures are illustrative in nature and not intended to limit the subject matter defined by the claims. The following detailed description can be understood when read in conjunction with the following drawings, where like structure is indicated with like reference numerals in which:
**FIG.** 1 illustrates a profile view of the tampon applicator according to the invention.;
**FIG.** 2 illustrates an isometric schematic partial cut of a component of the tampon applicator, specifically the barrel;
**FIG.** 3 illustrates a profile view of a component of the tampon applicator, specifically the fingergrip according to an embodiment of the invention;
**FIG.** 4 illustrates a profile view of a component of the tampon applicator, specifically the fingergrip according to another embodiment of the invention;
**FIG.** 5 illustrates a profile view of two components of the tampon applicator, specifically the fingergrip and the plunger.

### DETAILED DESCRIPTION OF THE INVENTION

The present invention concerns a tampon applicator. The invention also concerns a tampon assembly comprising said tampon applicator as well as the process for making such tampon applicator.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

As used herein, the following terms have the following meanings:
"A", "an", and "the" as used herein refers to both singular and plural referents unless the context clearly dictates otherwise. By way of example, "a component" refers to one or more than one component.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed invention. However, it is to be understood that the value to which the modifier "about" refers is itself also specifically disclosed.

"Comprise", "comprising," and "comprises" and "comprised of" as used herein are synonymous with "include", "including", "includes" or "contain", "containing", "contains" and are inclusive or open-ended terms that specifies the presence of what follows e.g. material and do not exclude or preclude the presence of additional, non-recited material, additives, lubricants, solvents, known in the art or disclosed therein; e.g. component and do not exclude or preclude the presence of additional, non-recited components, features, element, members, steps, known in the art or disclosed therein.

The recitation of numerical ranges by endpoints includes all numbers and fractions subsumed within that range, as well as the recited endpoints except where otherwise explicitly stated by disclaimer and the like.

As used herein, the terms "inward" or "inwardly" and "outward" or "outwardly" are in reference to the generally cylindrical shape of the tampon applicator and each components of said applicator according to the invention. The main components of a tampon applicator, which are the barrel, the fingergrip and the plunger, are substantially cylindrical defining with their respective housing, or wall, of given circumference an interior space (inside), or cavity, and an exterior space (outside). "Inward" and "inwardly" refer to an element extending at least partially radially towards the centre of the cylindrical shape of said component whereas "outward" and "outwardly" refer to an element extending at least partially radially away from the centre of the cylindrical shape of said component. In other words, "inward" means directed toward the interior and situated inside the cylindrical shape whereas "outward" means directed toward the outside or away from a centre and situated outside the cylindrical shape.

As used herein, the terms "outer surface" or "exterior surface" and "inner surface" or "interior surface" are in reference to the generally cylindrical and hollow shape of the tampon applicator and each components of said applicator according to the invention. The main components of a tampon applicator, which are the barrel, the fingergrip and the plunger, are substantially cylindrical and hollow thereby defining with their respective wall, or housing, of given circumference an interior cavity (inside), or space, and an exterior space (outside). For each component, meaning barrel, fingergrip, plunger, the "outer surface" or "exterior surface" refers to the surface of the wall facing the outside space, or exterior, and the "inner surface" or "interior surface" refers to the surface of the wall facing the interior cavity. Similarly, the outer diameter is the diameter of the component defined by the wall, or housing, on the outer surface and the inner diameter refers to the diameter of the component defined by the wall on the inner surface.

The term "consisting essentially of" does not exclude the presence of additional materials which do not significantly affect the desired characteristics of a given composition or product. Exemplary materials of this sort would include, without limitation, pigments, antioxidants, stabilizers, surfactants, waxes, flow promoters, solvents, particulates and materials added to enhance processability of the composition.

The term "finished" or "final", when used with reference to a product, means that the product has been suitably manufactured for its intended purpose.

"Texture" as used herein refers to the structure, feel, and appearance of a component.

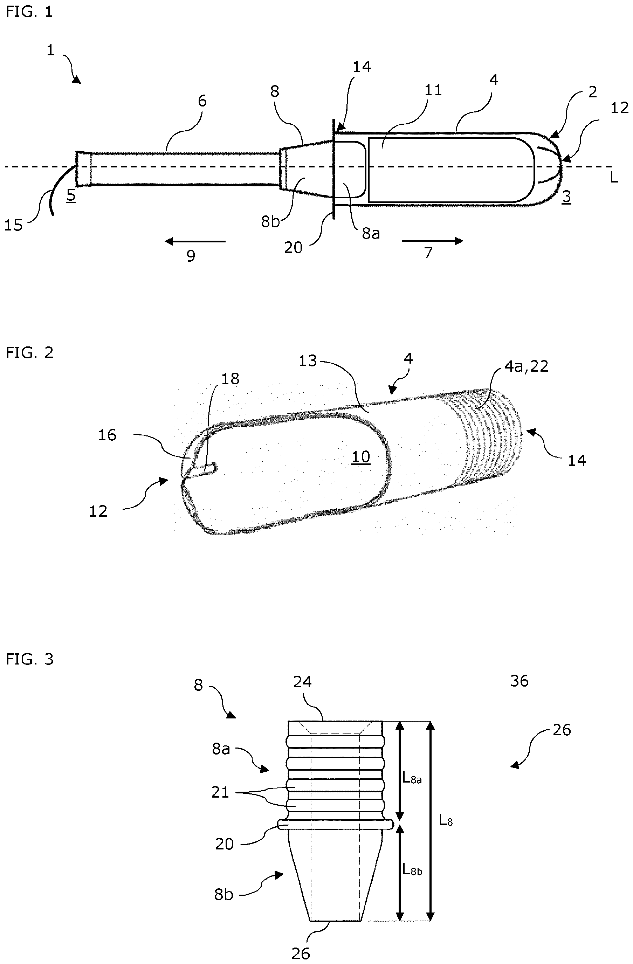

As used herein, the term "distal end" refers to the portions of the tampon applicator and of its parts that are most remote from the body of a user when a tampon pledget is being emplaced in a body cavity using said applicator. The term "proximal end" refers to the portions of the tampon applicator and of its parts that are closest to the body of the user when the tampon is being emplaced. Accordingly, the terms "proximal" or "proximally", and "distal" or "distally", as used herein, specify that a given portion or component of the tampon applicator or of its parts is relatively closer to, respectively, the proximal end or the distal end of the applicator or of its parts. Similarly, the terms "proximal direction" and "distal direction" respectively refer to the directions towards the proximal end or towards the distal end of the tampon applicator or of its parts, respectively. In FIG. 1, the proximal end and distal end of the tampon applicator 2 and its components, e.g. a barrel 4, a fingergrip 8 and a plunger 6, as meant herein are denoted by reference signs 3 and 5 respectively; and the proximal and distal directions as meant herein are denoted by reference arrows 7 and 9, respectively. In other terms, the proximal end 3 is the insertion or introduction end, arranged to be emplaced in a body cavity whereas the opposed distal end 5 is the withdrawal end used to withdraw the applicator from said body cavity.

Embodiments according to the disclosure will now be described. It is understood that technical features described in one or more embodiments maybe combined with one or more other embodiments without departing from the intention of the disclosure and without generalization therefrom.

FIG. 1 illustrates a cross-sectional view of a tampon assembly 1 comprising a tampon pledget 11 and a tampon applicator 2 according to the invention. The tampon applicator 2 comprises a barrel 4, also known as the outer tube, a plunger 6, also known as the ejector tube and a fingergrip 8. The barrel 4 and plunger 6, have a generally cylindrical shape. The barrel 4 preferably has a larger diameter than one end of the fingergrip 8, and the fingergrip 8 has a larger diameter than the plunger 6. The plunger 6 having a lesser diameter than the barrel 4 and fingergrip 8 is arranged in way that it can slide within the fingergrip 8 and within the barrel 4. In other words, the barrel 4 is dimensioned to fit closely and telescopically over a portion of the fingergrip 8. The plunger 6 is dimensioned and arranged to easily slide within the fingergrip 8 and barrel 4, with minimal clearance in between the plunger 6 and the fingergrip 8. The plunger 6 is hollow to allow proper placement of a withdrawal string 15 such as commonly provided at the withdrawal end, or proximal end, of tampon pledgets 11.

The barrel 4 is also hollow thus defining with its housing, or wall, an interior cavity 10 configured to store or contain a portion of the fingergrip 8, a portion of the plunger 6 and the tampon pledget 11 entirely. The barrel 4, the fingergrip 8 and the plunger 6 all extend in a longitudinal direction L. As illustrated in FIG.1, the tampon assembly 1 comprises a group of components arranged to fit closely one within another, we can also say that the barrel 4, fingergrip 8, pledget 6 and tampon pledget 11 are nested altogether.

The elongated barrel 4 has a discharge end 12 arranged at a first longitudinal extremity, more specifically at the proximal end 3 of the tampon applicator 2, and an open end 14 at the opposite longitudinal extremity, *i.e.* at the proximal end, or in other words at the longitudinal extremity opposite to the discharge end 12. The open end 14 has an opening to enable the introduction of the tampon pledget 11 as well as a portion of the fingergrip 8 and a portion of the plunger 6 during the process to manufacture the tampon assembly 1. As illustrated in FIG. 3, the barrel 4 comprises a main body section 13 arranged between the discharge end 12 and the open end 14. The plunger 6 is adapted to abut against the tampon pledget's 11 distal end and push it to expel said tampon pledget 11 through the discharge end 12 of the barrel 4.

As illustrated in FIG. 3, the distal discharge end 12 of the barrel 4 is formed with a plurality, e.g., two to sixteen, more commonly two to eight, and most commonly four or six petals 16 which are separated from each other by respective slots 18 or gap. The petals 16 are relatively flexible and are normally biased in a substantially arcuate closed configuration to form a rounded, or dome-shaped, tip having a central opening at the proximal end 3 of the tampon applicator 2. The rounded shape of the proximal end 3 helps facilitate the insertion of the tampon applicator 2 into a body cavity. The petals 16 are flexible and can be deformed by the tampon pledget 11 when it is being pushed by the plunger 6 and expelled through the discharge end 12 of the barrel 4.

According to the invention, the barrel 4 is formed from, or made out of, a material comprising cellulosic fibers, the plunger 6 is formed from a material comprising cellulosic fibers, and the fingergrip 8 is formed from a material comprising a mixture of cellulosic fibers and starch. Preferably, the barrel 4 and the plunger 6 are formed from the same material such as cardboard or paperboard or alternatively the barrel 4 and the plunger 6 can be formed from different material, for instance the barrel 4 can be formed from cardboard and the plunger 6 is formed from paperboard. By comprising a material comprising cellulosic fibers, the environment impact of the tampon applicator is lowered and is more environmentally friendly. Preferably the barrel 4 and the plunger 6 are formed from material consisting essentially of cellulosic fibers such as cardboard and/or paperboard. The basis weight of the barrel 4 is comprised between about 10 gsm to about 150 gsm (gram per square meter, g/m2), preferably between about 30 gsm to 120 gsm, more preferably between about 40 gsm and about 100 gsm. The basis weight of the plunger 6 is comprised between about 10 gsm to about 150 gsm (gram per square meter, g/m2), preferably between about 30 gsm to 120 gsm, more preferably between about 40 gsm and about 100 gsm.

The fingergrip 8 is formed from a material comprising a mixture of cellulosic fibers and starch. Such material has also the benefit of being more environmentally friendly than plastic while enabling more options in terms of processing and moulding to impart the finger grip with more gripping features than a conventional cardboard applicator. Indeed, cardboard while being eco-friendly, is a material that offers limited options in terms of shaping and moulding. Thus as explained above, conventional cardboard applicators are slippery and have limited gripping features. While plastics such a bio-plastic could improve the gripping features, in terms of sustainability, it is preferable to have a fingergrip comprising 100% natural elements such as cardboard and starch.

Preferably the fingergrip 8 is formed from a material comprising a mixture of cellulosic fibers, starch and optionally water or a water-based compound. Preferably, the material forming the fingergrip 8 comprises between 60 to 80 wt. % of starch, preferably between 65 to 75 wt.% of starch, more preferably about 70% of starch, between 8 to 25 wt.% of cellulosic fibers, preferably between 10 to 20 wt.% of cellulosic fibers, preferably paper fibers, and between 15 to 25 wt.% of water or water-based compound, preferably about 18 wt.% of such compound. Such material while being eco-friendly and sustainable, enables a proper moulding to shape and form the fingergrip as desired. It is also possible to incorporate other additives providing an additional function, such as disinfectants, cleaning agents or perfumes. Retention agents may also be added so as to improve the retention of starch in the sheet, in particular for sheets of low weight.

As illustrated in FIG. 3, the fingergrip 8 comprises a circumferential flange 20, or collar portion, extending, or projecting, outwardly from the outer circumferential surface of the fingergrip 8 in a radial direction, preferably beyond the outer circumference surface of the barrel 4 as illustrated in FIG. 1. In other terms, the diameter of the flange 20 is greater than the outer diameter of the barrel. Such flange 20 enables to have a proper grasping element for a user to grasp, or have a grip on, when applying a tampon. The flange 20 is arranged in a central portion of the finger grip 8 thereby dividing the fingergrip 8 in two portions, or parts, with a raised portion 8a and a tapered or curved portion 8b. In other words, the fingergrip 8 comprises a raised portion 8a, a flange 20 or collar portion, and a tapered or curved portion 8b, the flange 20 being arranged in-between the raised portion 8a and the tapered of curved portion 8b.

According to an embodiment, the fringergrip 8 comprises a raised portion 8a comprising a plurality of raised elements 21, or deformations, such as ribs 21, embossments or ledges. The plurality of deformations extend outwardly and/or inwardly, such as ribs, grooves, indentations, recess, protrusions, dots or any polygonal shape. As illustrated in FIG. 3, the raised elements 21 comprises circumferential rings extending radially outward meaning away from the interior cavity of the fingergrip. The raised elements 21 can be arranged in rows, parallel at uniform distant from one another. In the case of dots or any other polygonal shapes, the raised elements 21 can be arranged uniformly or in a staggered configuration. The plurality of raised elements 21 are preferably arranged on the exterior surface of the fingergrip 8. Although mentioned as a "plurality of raised elements", this terminology also encompasses lowered elements (oriented inward) and only one raised or lowered element.

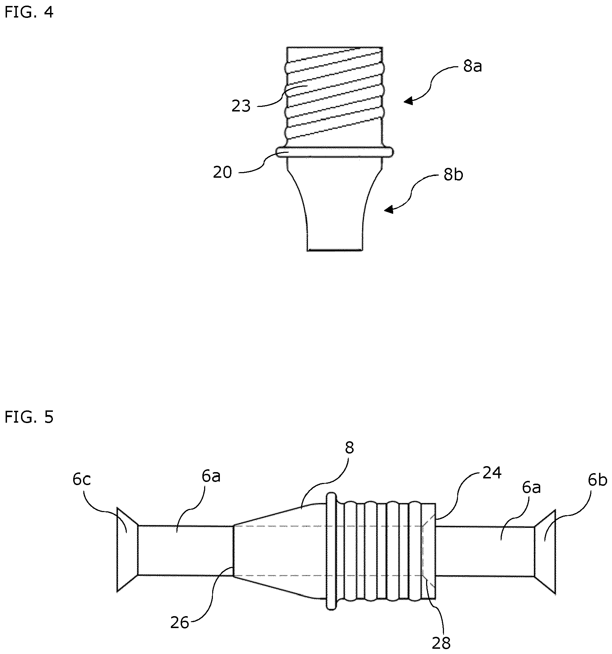

According to one particular embodiment as illustrated in FIG. 4, the fingergrip 8, in particular the raised portion 8a of the fingergrip 8, comprises a thread 23 arranged on the exterior surface of the fingergrip 8.

The raised portion 8a of the fingergrip 8 is configured to be inserted within the barrel 4 through the open end 14 of the barrel 4 as illustrated in FIG.1. In order words, when assembled, or in its final configuration, the tampon applicator 2 comprises a barrel 4, a fingergrip 8 and a plunger 6, the raised portion 8a of the fingergrip 8 is nested within the interior cavity 10 of the barrel whereas the tapered or curved portion 8b is arranged outside of the interior cavity 10. The open end 14 of the barrel 4 abuts against a side of the flange 20.

In order to ensure the structural integrity of the tampon applicator and that the raised portion 8a stays lodged within the interior cavity 10, the outer distal surface of the barrel 4 may also be provided with a raised portion comprising a ridged or knurled surface, for sake of clarity, this portion will be referred to as ridged portion 4a henceforth. In other words, the ridged portion 4a arranged at the distal end of the barrel 4 comprises a plurality of deformations 22 extending outwardly and/or inwardly, such as ribs, grooves, indentations, recess, protrusions, dots or any polygonal shape, to provide interlocking means with the raised portion 8a of the fingergrip. The plurality of deformations 22 can comprise circumferential ribs, or rings, extending radially outward away from the interior cavity 10. The plurality of deformations 22 can be arranged in rows, parallel at uniform distant from one another. In the case of dots or any other polygonal shapes, the plurality of deformations 22 can be arranged uniformly or in a staggered configuration. The plurality of deformations 22 are preferably arranged on the interior surface of the barrel 4. Said deformations 22 can also provide additional anti-slip features to the tampon applicator 2. Although mentioned as a "plurality of deformations", this terminology also encompasses only one deformation.

As explained previously, the fingergrip 8, in particular the raised portion 8a of the fingergrip 8 and the barrel 4, in particular the ridged portion 4a of the barrel 4 comprise interlocking means connecting the fingergrip 8 to the barrel 4. As described above, the interlocking means can be a plurality of ribs where ribs and grooves are arranged in succession. In particular, the interlocking means comprises the plurality of raised elements 21 arranged on the fingergrip 8 and the plurality of deformation 22 arranged on the barrel 4. Once assembled, the ribs arranged in the raised portion 8a of the fingergrip 8 rest in the grooves of the ridged portion 4a of the barrel 4 and the ribs 21 of the ridged portion 4a of the barrel 4 rest in the grooves of the raised portion 8a of the fingergrip 8 thereby ensuring an annular connection between the two components of the tampon applicator.

According to the embodiment illustrated in FIG. 4 where the fingergrip 8 comprises a thread 23 arranged at the outer surface of the fingergrip 8 in the raised portion 8a, the barrel 4, namely the ridged portion 4a at the proximal end of the barrel 4, also comprises a thread arranged on the interior surface of the barrel 4, meaning on the surface, or side, of the housing facing the interior cavity 10. The threads on the barrel 4 and the fingergrip 8 are of complementary shape, meaning with the same height and pitch, to ensure a screwing connection between the fingergrip 8 and the barrel 4.

The invention is not limited to those embodiments, other interlocking means can be arranged to ensure the connection between the fingergrip 8 and the barrel 4, for instance, the connection can be ensured by a cantilever snap fit connection, where the fingergrip 8 comprises a clip with protruding teeth and the barrel 4 comprises holes to receive said teeth. The connection can be ensured by a bayonet connection where the fingergrip 8 comprises at least one pin and the barrel 4 comprises at least one corresponding L-shaped slot, Γ-shaped slot (L with a third small branch at the extremity), U-shaped slot or any adapted cam track to ensure a fixation of the pin. Depending on the manufacturing process considered, a connection type can be privileged over the other.

According to the embodiment illustrated in FIG. 3 or FIG. 4, the fingergrip comprises a tapered portion 8b (FIG. 3) or curved, or concave, portion 8b (FIG. 4) arranged between the flange 20 and one end, or extremity of the fingergrip 8, wherein the diameter, namely the outer diameter of the fingergrip 8 decreases from the flange 20 to said one end. The inner diameter of the fingergrip 8 is constant throughout the length of the fingergrip 8. The inner diameter of the fingergrip 8 is generally slightly greater than the outer diameter of the plunger 6 to ensure the proper sliding of said plunger 6.The tapered or curved portion 8b provides an additional gripping surface for the user. The fingergrip 8 being formed from a material comprising a mixture of starch and cellulosic fibers, it is more abrasive than cardboard thereby offering anti-slip features and the user can perceive a different texture between the fingergrip 8 in comparison to the barrel 4 or plunger 6. Alternatively, it is possible to add one or more natural friction reducing agents to render the surface smoother, given that there is already a circumferential flange 20 that can be grasped.

As illustrated in these drawings, the fingergrip 8 comprises a tapered portion or curved portion 8b arranged on one side of the flange 20 and a raised portion 8a arranged on the opposite side of the flange 20. More precisely, the fingergrip 8 comprises a proximal end 24 and a distal end 26. The flange 20 is arranged between said proximal end 24 and a distal end 26 of the fingergrip 8, preferably at a central position, more preferably at the center in terms of length (or height). The raised portion 8a is arranged between the proximal end 24 of the fingergrip 8 and the flange 20 and the tapered or curved portion 8b is arranged between the flange 20 and the distal end 26 of the fingergrip 8.

Simply put, the fingergrip 8 comprises a flange 20 with two portions 8a,8b each arranged on one side of the flange 20, the portions 8a,8b being different in terms of features, shape, dimensions and/or material.

According to an embodiment, the fingergrip 8, preferably the raised portion 8a of the fingergrip 8, comprises a generally conical shape. In other words the outer diameter of the fingergrip 8, and raised portion 8a, decreases from the flange 20 to the proximal end 24. Such embodiment can facilitate the connection between the interlocking means.

The fingergrip 8 also extends in the longitudinal direction L of the tampon applicator 2. The fingergrip 8 has a length Ls which can be comprised between 20 and 50 mm, preferably between 25 and 40 mm, more preferably between 30 and 35 mm. The flange 20 is arranged at the central position of the fingergrip 8. According to a specific embodiment, the flange 20 is arranged at the center, in order words the distance between the proximal end 24 of the fingergrip 8 and the flange 20 is equal to the distance between the distal end 26 of the fingergrip 8 and the flange 20. Or in other words, the distance between one of the end 24,26 of the fingergrip 8 and the flange 20 is equal to Ls/2, the raised portion 8a and the tapered or curved portion 8b extending on a same distance.

The raised portion 8a has a length Lsa and the tapered or curved portion 8b has a length L_{8b} as illustrated in FIG. 3 (L₈ₐ=L_{8b}). According to one embodiment, the length Lsa of the raised portion 8a is equal to the length L_{8b} of the tapered or curved portion 8b. Alternatively, the length L8a of the raised portion 8a can be greater than the length L8b of the tapered or curved portion 8b (L₈ₐ>L_{8b}). It is not preferable that the length L_{8b} be greater than the length Lsa as it would imply either that the length L₈ₐ of the raised portion 8a is reduced and thus the connection between the fingergrip 8 and the barrel 4 would be deteriorated or that the length L_{8b} of the tapered or curved portion 8b is too great and the plunger 6 could not properly expel the tampon pledget 11. As we will see hereunder, the fingergrip 8 also serves as an abutment element for the plunger 6. Specifically, when applying a tampon, the distal end 26 of the fingergrip 8 serves as a retention mean for the plunger 6 where the distal end of the plunger 6 abuts against the distal end 26 of the fingergrip 8. Hence by increasing the distance L_{8b}, the plunger 6 has to travel a greater distance to ensure a satisfactory expulsion of the tampon through the discharge end 12. Of course, if it is a specific objective to have additional gripping features on the fingergrip, it is possible to increase the length L_{8b} of the tapered or curved portion 8b (L_{8b}>L₈ₐ) but that could imply increasing the length of the plunger.

As illustrated in FIG. 5, the fingergrip 8 preferably serves as a retention means for the plunger 6. The plunger 6 may comprises an elongated cylindrical or tubular portion 6a, an outwardly directed circumferential retention flange 6b at its proximal end and/or an outwardly directed circumferential retention flange 6c at its distal end. Similarly, the plunger 6 may comprises a flared portion at its proximal and/or distal ends. The retention flanges 6b,6c control the extent of insertion or withdrawal of the plunger 6 respectively in or out the barrel 4 of the tampon applicator 2. Specifically, the retention flange 6b at the proximal end of the plunger 6 is arranged to abut against the proximal end 24 of the finger grip 8 when pulling the plunger in the distal direction 9 and the retention flange 6c at the distal end of the plunger 6 is arranged to abut against the distal end 26 of the finger grip 8 when pushing the plunger 6 in the proximal direction 7. The retention flange 6b at the proximal end and/or the retention flange 6c at the distal end may comprise a chamfered or sloped cross section. As illustrated in FIG. 5, the proximal end 24 of the fingergrip 8 comprises a shoulder portion 28, or a bottleneck portion, that can be chamfered and is arranged to receive the retention flange 6b of the plunger. In other words, the inner surface of the fingergrip 8 at its proximal end 24 is of complementary shape as the retention flange 6b at the proximal end of the plunger.

According to an embodiment, the flange 20 has a diameter that is at least 2 mm greater than the outer diameter of the barrel 4. This ensures that the flange protrudes sufficiently from the tampon applicator and can be grasped more easily.

For instance, the circumferential flange 20 has a diameter comprised between 10 and 60 mm, preferably between 11 and 40 mm, more preferably between 11 and 25 mm. Of course tampons depending on their absorbency level can vary in diameter having a nominal diameter ranging from 8 to 20 mm, hence the minimum diameter of the barrel ranges from 9 to 21 mm.

A "Super Plus" absorbency tampon should have a total absorbency as measured by the industry standard Syngyna test of 12-15 grams. These tampons pledgets 11 have a higher dry width, comprised between about 15 mm and 20 mm and therefore applicators 2 for such tampons usually have a barrel 4 with a greater outer diameter comprised between 15 and 21 mm. Hence for such tampons applicators 2, the flange 20 has a diameter comprised between 16 mm and 60 mm, preferably between 17 mm and 40 mm, preferably between 17 mm and 25 mm.

A "Super" absorbency tampon should have a total absorbency as measured by the Syngyna test of 9-12 grams for tampon pledgets 11 usually with a dry width comprised between about 13 and 15 mm. The applicator for such tampons usually has a barrel 4 with an outer diameter comprised between 13 and 16 mm, hence for such tampon applicator 2, the flange 20 has a diameter comprised between 14 mm and 55 mm, preferably between 15 mm and 35 mm, preferably between 15 mm and 25 mm.

A "Regular" absorbency tampon should have a Syngyna absorbency of 6-9 grams for tampon pledgets 11 usually with a dry width comprised between about 10 and 13 mm. The applicator for such tampons usually has a barrel with an outer diameter comprised between 10 and 14 mm, hence for such tampons applicator 2 the flange 20 has a diameter comprised between 11 mm and 50 mm, preferably between 12 mm and 30 mm, preferably between 12 mm and 25 mm.

A "Junior" absorbency tampon should have a Syngyna absorbency of less than 6 grams for tampon pledgets 11 usually with a dry width comprised between about 8 and 10 mm. The applicator for such tampons usually has a barrel with an outer diameter comprised between 9 and 11 mm, hence for such tampons applicator the flange has a diameter comprised between 10 mm and 40 mm, preferably between 11 mm and 30 mm, preferably between 11 mm and 25 mm.

To summarize, the diameter of the flange 20 is 1.1 to 5 times greater than the outer diameter of the barrel 4, preferably the diameter of the flange 20 is 1.2 to 3 times greater than the outer diameter of the barrel 4, more preferably the diameter of the flange 20 is 1.2 to 2.5 times greater than the outer diameter of the barrel 4. This ensures that the flange protrudes sufficiently from the tampon applicator and can be grasped more easily.

The flange 20 can have a round circular circumference, or shape or profile. The flange 20 can also have an elliptical circumference, or shape or profile.

The diameter of the plunger 6, specifically the outer diameter of the tubular portion 6a of the plunger 6, is usually around 6 to 8 mm, preferably about 7 mm for any given absorbency level. Hence, based on the dimensions listed hereabove the diameter of the flange 20 is 1.5 to 10 times greater than the outer diameter of the plunger 6, preferably the diameter of the flange 20 is 1.6 to 5 times greater than the outer diameter of the plunger 6, more preferably the diameter of the flange 20 is 1.7 to 3 times greater than the outer diameter of the plunger 6.

The invention also pertains to an assembly comprising a tampon applicator 2 according to any of the preceding claims and a tampon pledget 11 comprising absorbent material arranged within the barrel 4 of the tampon applicator 2. The invention also pertains to the use of a material comprising a mixture of cellulosic fibers and starch to form a fingergrip 8 component of a tampon applicator 2, said applicator comprising a circumferential flange 20 for providing an environmentally friendly applicator with improved gripping features.

The invention also pertains to a method to manufacture a tampon applicator 2 comprising at least the following steps:
- providing a cardboard shell comprising at least the barrel 4 comprising a portion, or an element, of the interlocking means such as ribs, grooves, holes, or thread ;
- molding the material comprising a mixture of cellulosic fibers, or cardboard, and starch, into the fingergrip 8, said fingergrip 8 comprising a gripping feature, preferably the flange 20, and another portion, or complementary element, of the interlocking means such as ribs, grooves, clips, or thread;
- assembling the fingergrip 8 and the barrel 4 by connecting the interlocking means together.

The tampon pledget 11 can be introduced within the barrel 4 interior cavity 10 prior to the connection of the interlocking means. In this case, the tampon pledget is loaded through the open end 14 of the barrel. Alternatively, the tampon pledget 11 can be introduced within the barrel 4 interior cavity 10 after the connection of the interlocking means. In this case the tampon pledget 11 is loaded through the discharge end 12, followed by the closing of the petals 16. Similarly, the plunger 6 can be introduced with the fingergrip 8 interior space prior to or after the assembly of the fingergrip 8 and the barrel 4 through the proximal end 24 or the distal end 26 of the fingergrip 8.

It is supposed that the present invention is not restricted to any form of realization described previously and that some modifications can be added to the presented example of fabrication without reappraisal of the appended claims.

## Claims

1. Tampon applicator (2) comprising at least three separate components selected from a barrel (4), a plunger (6) and a fingergrip (8), wherein
- the barrel (4) is formed from a material comprising cellulosic fibers,
- the plunger (6) is formed from a material comprising cellulosic fibers, and
- the fingergrip (8) is formed from a material comprising a mixture of cellulosic fibers and starch.

2. Tampon applicator (2) according to claim 1, wherein the material comprising cellulosic fibers is cardboard or paper and/or the material comprising a mixture of cellulosic fibers and starch is a mixture of cardboard or paper and starch derived from natural sources such as corn, wheat, rice, tapioca, potato, and/or maize.

3. Tampon applicator (2) according to claim 1 or 2, wherein the material forming the fingergrip (8) comprises between 60 to 80 wt. % of starch, preferably between 65 to 75 wt.% of starch, more preferably about 70% of starch, and between 8 to 25 wt.% of cellulosic fibers, preferably between 10 to 20 wt.% of cellulosic fibers.

4. Tampon applicator (2) according to any of the preceding claims, wherein the fingergrip (8) comprises a flange (20) projecting from an outer surface of the fingergrip (8) in a radial direction preferably beyond an outer surface of the barrel (4).

5. Tampon applicator (2) according to claim 4, wherein the flange (20) is arranged in a central portion of the fingergrip (8).

6. Tampon applicator (2) according to any of the preceding claims, wherein the fingergrip (8) and the barrel (4) comprise interlocking means connecting the fingergrip (8) to the barrel (4).

7. Tampon applicator (2) according to any of the preceding claims, wherein the fringergrip (8) comprises a raised portion (8a) comprising a plurality of raised elements (21) such as ribs, embossments or ledges arranged on the exterior surface of the fingergrip (8).

8. Tampon applicator (2) according to any of the preceding claims, wherein the fingergrip (8) comprises a thread (23) arranged on the exterior surface of the fingergrip (8).

9. Tampon applicator (2) according to claim 8, wherein the barrel (4) comprises a thread arranged on the interior surface of the barrel (4) of complementary shape as the thread (23) on the fingergrip (8).

10. Tampon applicator (2) according to any of the claims 5 to 9 dependent of claim 4, wherein the fingergrip (8) comprises a tapered or curved portion (8b) arranged between the flange (20) and one end (26) of the fingergrip (8), wherein the outer diameter of the fingergrip (8) decreases from the flange (20) to said one end (26).

11. Tampon applicator (2) according to any of the claims 7 to 9 and claim 10, wherein the fingergrip (8) comprises the tapered or curved portion (8b) arranged on one side of the flange (20) and a raised portion (8a) arranged on the opposite side of the flange (20).

12. Tampon applicator (2) according to any of the claims 4 to 11, wherein the diameter of the flange (20) is at least 2 mm greater than the outer diameter of the barrel (4).

13. Tampon applicator (2) according to any of the preceding claims, wherein the fingergrip (8) comprises a shouldered portion (28) on the interior surface of the fingergrip (8).

14. Assembly comprising a tampon applicator (2) according to any of the preceding claims and a tampon pledget (11) comprising absorbent material arranged within the barrel (4) of the tampon applicator (2).

15. Use of a material comprising a mixture of cellulosic fibers and starch to form a fingergrip (8) component of a tampon applicator (2), said applicator comprising a circumferential flange (20) for providing an environmentally friendly applicator with improved gripping features.
